# EUROPEAN PATENT SPECIFICATION

(11) **EP 2 944 314 B1**
(45) Date of publication and mention of the grant of the patent: **02.09.2020**
(21) Application number: 14737496.1
(22) Date of filing: 13.01.2014
(51) Int. Cl.: A61K 35/63, A61K 33/06, A61P 19/02, A61K 33/10, A61L 27/36

(54) **PREPARATION FOR TREATING ARTHRITIS AND ARTHROSIS**
ZUBEREITUNG ZUR BEHANDLUNG VON ARTHRITIS UND ARTHROSE
PRODUIT POUR TRAITER LES ARTHRITES ET LES ARTHROSES

(30) Priority: 11.01.2013 RU 2013100994
(43) Date of publication of application: 18.11.2015
(73) Proprietor: Obshestvo S Ogranichennoj Otvetstvennostju "PARAFARM", Penza 440026 (RU)
(72) Inventor: STRUKOV, VILLORIJ Ivanovich, g. Penza 440011 (RU); PROKHOROV, Mikhail Dmitrievich, g. Penza 440011 (RU); JONES-STRUKOVA, Olga, Fort Worth, TX 76107 (US); TRIFONOV, Vjacheslav Nikolaevich, Penzenskaya obl. g. Zarechny 442960 (RU); ELISTRATOVA, Julija Anatolievna, g. Penza 440026 (RU); ELISTRATOV, Konstantin Gennadievich, g. Penza 440039 (RU); KURUS`, Natal`ja Vjacheslavovna, g. Penza 440026 (RU); EREMINA, Natalya Vyacheslavovna, g. Penza 440047 (RU); MAKSIMOVA, Marina Nikolaevna, g. Penza 440044 (RU); GALEEVA, Ramziya Timurshovna, g. Penza 440062 (RU); RADCHENKO, Larisa Grigorievna, g. Penza 440062 (RU); FEDOROV, Aleksandr Viktorovich, Penzenskaya obl. g. Kuznetsk 442530 (RU); KRUTYAKOV, Evgeny Nikolaevich, g. Penza 440062 (RU); ANDREEVA, Elena Stanislavovna, g. Penza 440062 (RU); ELISTRATOVA, Tatiana Viktorovna, g. Penza 440062 (RU); KHOMYAKOVA, Irina Vladimirovna, g. Penza 440062 (RU); TOLBINA, Galina Anatolievna, g. Penza 440444 (RU)
(74) Representative: Engel, Christoph Klaus
(86) International application number: PCT/RU2014/000009
(87) International publication number: WO 2014/109678

(56) References cited:
- WO-A1-2012/166003
- WO-A2-2013/006094
- BE-A6- 1 015 783
- RU-C1- 2 268 734
- RU-C1- 2 412 616
- US-A1- 2005 233 007
- STRUKOV V: "Mirovoe otkrytie v borbe s perelomami i osteoporozom! [World discovery in the fight against osteoporosis and fractures]", POLIKLINIKA, RU, no. 5, 1 May 2012 (2012-05-01), pages 126-127, XP008180115, ISSN: 2311-2441
- STRUKOV V.: 'Mirovoe otkrytie v borbe s perelomami i osteoporozom!' POLIKLINIKA May 2012, pages 126 - 127, XP008180115

## Description

Invention relates to medicine, and particularly, to compositions for use in methods of treatment of arthritis and osteoarthrosis.

There is known a phytotherapy medicament "Alevalon" for treatment of arthritis and method of its preparation (2102998, February 10, 1995). A medicament for treatment of arthritis and method of its preparation relate to compositions which contain substances from plants, and can be used as a base for remedy for treatment of mono- and polyarthritis, and osteoarthrosis. The composition comprises ethanolic extract of following ingredients in following ratio (in parts by weight): roots of Inula helenium - 2-5 parts, roots of Comarum palustre - 1-4 parts, buds of birch (Betula), the rest - ethanol. The composition can also include ethanolic extract of pantocrine or ginseng. The method comprises alternate introduction of the above mentioned components in solution, including its heating and periodic shaking.

The main disadvantages of this known composition and method of its preparations are that they in common are aimed to the treatment of arthritis and don't concern the problem of osteoarthrosis.

STRUKOV, V. "Mirovoe otkrytie v borbe s perelomami I osteoporozom! [World discovery in the fight against osteoporosis and fractures]", POLIKLINIKA, RU, no. 5,1 May 2012 (2012-05-01), pages 126-127, XP008180115, ISSN: 2311-2441, shows a composition called "Osteomed" comprising drone brood and calcium citrate. The composition is used to close bone cavities in osteoporosis and in rheumatology as the bone cavities are underlaying cause of arthritis and arthrosis.

WO 2012/166003 A1 describes a composition for filing bone cavities. The composition contains drone brood and calcium.

WO 2013/006094 A2 discloses a composition for the treatment of osteoarthritis and osteoarthrosis. The composition comprises drone brood lyophilizate, dihydroquercetine powder and ground dandelion root powder.

The closest to the above mentioned technical solution is method of treatment of rheumatoid arthritis (RU 2 268 734, June 21, 2004). Invention relates to medicine, and particularly, to rheumatology, and aimed to treatment of any forms and phases of rheumatoid arthritis (RA). Method includes base immunomodulatory and symptomatic therapies, and, additionally, treatment by composition Calcium-D3 with adjustment of dose, duration, and multiplicity of treatment courses depending the phase, degree, characteristics of course of disease, and bone mineral density (BND).

The main disadvantage of the above mentioned method of treatment is the high risk of development of hypercalciemia, as well as that this method is not aimed to the treatment of osteoarthrosis. The more detailed description of treatment of hypercalciemic states gives Prof. V.I.Strukov in his book "Disorders of calcium metabolism. Hypercalciemic states" / Ed. by Penza Institute of physician's postgraduate training. Penza, "Rodnoi dom", 2011.

Technical result to which is aimed claimed invention is to obtain the closing of cavities in bony tissue and to accelerate the recovery of patients having arthritis and osteoarthrosis.

The scope of the protection sought is defined by the claims. This technical result is being obtained by administering of drone brood and calcium compound. According to the invention, drone brood and calcium compound should enter into the organism simultaneously within 24 hours. Calcium compound and drone brood for treatment of arthritis and osteoarthrosis may be in form of form of powder, tablets or capsules, wherein calcium compound is one from the following group of compounds or any combination of them - calcium carbonate, calcium citrate, calcium gluconate, calcium aspartate, calcium ascorbate, calcium aminochelate, calcium fumarate, calcium succinate, and calcium phosphate and calcium citrate. Osteoarthrosis and arthritis, internationally known as osteoarthritis, are widespread group of diseases of various origins, for which are characteristic development of dystrophic and inflammatory changes in bony tissue.

The most widespread today in CIS countries are some dangerous and inefficient programs of treatment of "polyarthritis" with steroid and non-steroid antiinflammatory and analgetic medicaments which assure only symptomatic effect. These methods have a series of important disadvantages because they don't pay attention to senile causes of arthritis and osteoarthrosis.

We have ascertained that the cause of senile osteoarthrosis and/or arthritis is presence of cavities in bony tissue. As a result, process of building of bony tissue is being felt behind the process of its destruction by osteoclasts.

Formed thereby bone cavities worsen normal metabolism of that bone part which is located behind cavity. As a result, when osteoclast destroys a bone cell, bone mineral matrix can do with it nothing, since the bone cavity do not allow it to be excreted from bony tissue. Therefore, the bone mineral matrix is excreted by organism from the bone. The result of its process is osteoarthrosis. Thus, the cause of osteoarthrosis is not consequence of age-related changes in muscle frame or way of stabilizing of human skeleton. The cause of osteoarthrosis consists in bone cavities which are hampering excretion of destroyed bone mineral matrix from the bones.

The cause of development of arthritis is the same: inflammatory processes in bony tissue. The bone cavity hampers the excretion of waste products of bone cells and destroyed cells from bony tissue. It results in inflammation - arthritis.

We have established during treatment of osteoporosis by "Osteomed" supplement that the use of drone brood allows closing of bone cavities. As soon as bone cavities begin to close, arthritic and osteoartotic processes also begin to stop and to turn backwards.

The speed of closing of bone cavities depends on the degree of calcium absorption by organism and may be increased with sufficient intake of calcium.

We have also established that even one-month administering of drone brood, plus diet with a great amount of calcium, gives a positive results: in patients treated with these products are disappeared aching pains in bones that allowed them to give up the use of nonsteroid anti-inflammatory remedies.

Further administering of the claimed composition leads to more closing of bone cavities thereby improving metabolism of bony tissue located behind bone cavity. Progression of the disease stops, joint mobility improves, pains during motion decrease.

The practice of using of the claimed composition in more than 1000 patients with osteoarthrosis and arthritis demonstrated significant progress in therapy after 6-9 months of treatment. Patients which earlier could not move because of severe pains began to walk and serve themselves.

During experimental confirmation drone brood was administered to patients orally in form of powder, tablets or capsules, both as separate acting agent as well as in combination with calcium compounds.

Depending on individual features of organism, amount of calcium taken with food, quantity and dimensions of bone cavities, the positive result may be achieved faster or slower, but it was obtained in all cases. As showed the practice, the practically significant results were obtained after long enough administering (9 months) in 100% of patients.

## Claims

1. A composition for use in the treatment of senile arthritis and osteoarthrosis, where the composition comprises drone brood and calcium compounds, wherein the calcium compound is one from the following group of compounds or any combination of them: calcium carbonate, calcium citrate, calcium gluconate, calcium aspartate, calcium ascorbate, calcium aminochelate, calcium fumarate, calcium succinate, and calcium phosphate.

2. The composition for use in the treatment of senile arthritis and osteoarthrosis according to claim 1, wherein the composition is made in a form of powder, tablets or capsules.

3. The composition for use in the treatment of senile arthritis and osteoarthrosis according to claim 1 or 2, wherein the drone brood and calcium compounds enter into the organism simultaneously within 24 hours.

## Patentansprüche

1. Zusammensetzung zur Verwendung bei der Behandlung von seniler Arthritis und Osteoarthrose, wobei die Zusammensetzung Drohnenbrut und Calciumverbindungen enthält, wobei die Calciumverbindung eine Verbindung aus der folgenden Gruppe von Verbindungen oder eine Kombination davon ist: Calciumkarbonat, Calciumcitrat, Calciumgluconat, Calciumaspartat, Calciumascorbat, Calciumaminochelat, Calciumfumarat, Calciumsuccinat, und Calciumphosphat.

2. Zusammensetzung zur Verwendung bei der Behandlung von seniler Arthritis und Osteoarthrose nach Anspruch 1, wobei die Zusammensetzung in Form von Pulver, Tabletten oder Kapseln hergestellt ist.

3. Zusammensetzung zur Verwendung bei der Behandlung von seniler Arthritis und Osteoarthrose nach Anspruch 1 oder 2, wobei die Drohnenbrut und Calciumverbindungen innerhalb von 24 Stunden gleichzeitig in den Organismus eintreten.

## Revendications

1. Composition pour son utilisation dans le traitement de l'arthrite sénile et de l'arthrose, où la composition comprend du couvain de faux-bourdon et des composés du calcium, dans laquelle le composé du calcium est l'un du groupe de composés suivant ou toute combinaison de ceux-ci : carbonate de calcium, citrate de calcium, gluconate de calcium, aspartate de calcium, ascorbate de calcium, aminochélate de calcium, fumarate de calcium, succinate de calcium, et phosphate de calcium.

2. Composition pour son utilisation dans le traitement de l'arthrite sénile et de l'arthrose selon la revendication 1, dans laquelle la composition est mise sous la forme de poudre, de comprimés ou de capsules.

3. Composition pour son utilisation dans le traitement de l'arthrite sénile et de l'arthrose selon la revendication 1 ou 2, dans laquelle le couvain de faux-bourdon et les composés du calcium entrent dans l'organisme simultanément en 24 heures.
